# EUROPÄISCHE PATENTSCHRIFT

(11) **EP 1 831 280 B1**
(45) Veröffentlichungstag und Bekanntmachung des Hinweises auf die Patenterteilung: **23.07.2008**
(21) Anmeldenummer: 05825088.7
(22) Anmeldetag: 10.12.2005
(51) Int. Cl.: C08G 18/78, C07C 273/18

(54) **VERFAHREN ZUR HERSTELLUNG VON FARBLOSEN, LAGERSTABILEN BIURETGRUPPENHALTIGEN POLYISOCYANATEN**
METHOD FOR PRODUCING COLOURLESS POLYISOCYANATES THAT CONTAIN BIURET GROUPS AND ARE STABLE IN STORAGE
PROCEDE POUR PRODUIRE DES POLYISOCYANATES INCOLORES, STABLES AU STOCKAGE ET CONTENANT DES GROUPES BIURET

(30) Priorität: 13.12.2004 DE 102004060122; 13.12.2004 DE 102004060123; 13.12.2004 DE 102004060121; 13.12.2004 DE 102004060120; 13.12.2004 DE 102004060131; 15.12.2004 DE 102004060739
(43) Veröffentlichungstag der Anmeldung: 12.09.2007
(73) Patentinhaber: BASF SE, 67056 Ludwigshafen (DE)
(72) Erfinder: WAGNER, Eva, 67346 Speyer (DE); BEY, Oliver, 67150 Niederkirchen (DE); WÖLFERT, Andreas, 74906 Bad Rappenau (DE); JÄHME, Joachim, 67240 Bobenheim-Roxheim (DE); BRUCHMANN, Bernd, 67251 Freinsheim (DE); BAYER, Alexander, 67117 Limburgerhof (DE)
(86) Internationale Anmeldenummer: PCT/EP2005/013276
(87) Internationale Veröffentlichungsnummer: WO 2006/063750

(56) Entgegenhaltungen:
- EP-A- 0 716 080
- WO-A-98/07771
- DE-A1- 2 918 739
- DE-A1- 19 525 474

## Beschreibung

Die vorliegende Erfindung betrifft ein Verfahren zur Herstellung von farblosen, lagerstabilen biuretgruppenhaltigen Polyisocyanaten aus Di- oder Polyisocyanaten mit Wasser und/oder Wasserdampf als Biuretisierungsmittel, und eine Vorrichtung dazu und deren Verwendung.

DE-C1 197 07 576 beschreibt ein Verfahren zur Herstellung von biuretgruppenhaltigen aromatischen Polyisocyanaten aus Isocyanaten und Diaminen, in dem Diamin und Isocyanat in einer Mischkammer miteinander zur Reaktion gebracht werden und anschließend in einer einstufigen Rührkessel oder gegebenenfalls auch einer mehrstufigen Rührkesselkaskade ausreagiert werden.

EP-B1 3505 beschreibt ebenfalls ein ähnliches Verfahren zur Herstellung von biuretgruppenhaltigen Polyisocyanaten aus Isocyanaten und Diaminen bei der die Aminkomponente mittels einer Glattstrahldüse in das Isocyanat eindosiert wird.

Nachteilig an diesen beiden Verfahren ist, daß sie von Aminen und Isocyanaten als Edukten ausgehen. Durch die hohe Reaktivität von Aminen ist die Reaktion mit dem Isocyanat schwer zu kontrollieren und kann so zur verstärkten Bildung unerwünschter Harnstoffe führen, die im Reaktionsmedium in der Regel schwer löslich sind und zu Verstopfungen führen können. Weiterhin laufen diese Verfahren in der Praxis bei relativ hohen Drücken und Temperaturen ab, was die Farbzahl der Produkte aufgrund der thermischen Belastung erhöht.

EP-A1 716 080 beschreibt ein Verfahren zur Herstellung von biuretgruppenhaltigen Isocyanaten aus Isocyanaten und Wasser oder Wasserdampf, wobei das Wasser zur Steuerung der Reaktion in feindisperser Fom eingebracht wird.

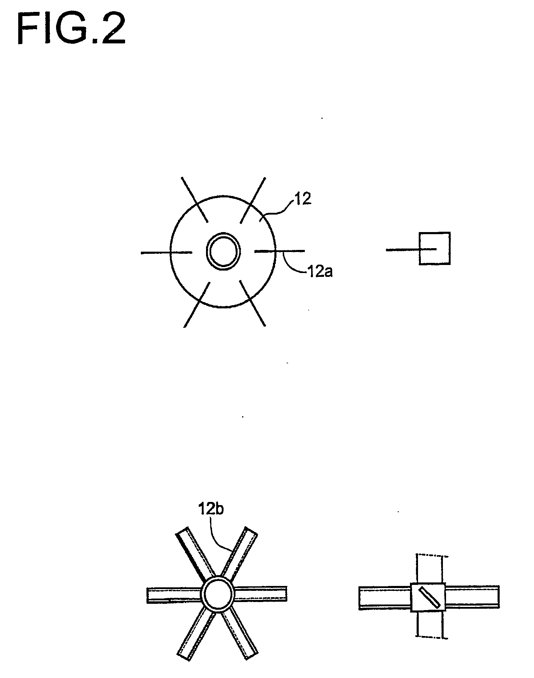

DE-A1 195 25 474 beschreibt ein Verfahren zur Herstellung von biuretgruppenhaltigen Isocyanaten aus Isocyanaten und Wasser oder Wasserdampf in einer kaskadierten Reaktoranordnung und im Gegenstrom. Gemäß der dort in Fig. 2 dargestellten Ausführungsform wird der Katalysator am Kopf des kaskadierten Reaktors und Wasserdampf als Biuretisierungsmittel am Boden desselben über ein Tauchrohr (6A in Fig. 3 der DE-A1 195 25 474) zugegeben.

Nachteilig an dieser Reaktionsführung ist, daß Wasserdampf über den Produktaustrag am Boden des Reaktors in nachgeschaltete Anlagenteile gelangen und dort zu einer unkontrollierten Reaktion unter Bildung von Polyharnstoffen führen kann. Ein so angebrachtes Tauchrohr neigt zudem zu Verstopfung infolge der Bildung von Inkrustierungen. Ferner muß durch diese Reaktionsführung die gesamt Dispergierungsenergie durch Rühren, d.h. über bewegte Bauteile in das Reaktionsgemisch eingebracht werden, was zu einer erhöhten Störanfälligkeit solcher bewegter Bauteile führt. Bewegte Bauteile erfordern wiederum Abdichtungen und Lagerungen, die, wie Tauchrohre, im laufenden Betrieb nicht oder nur schwierig auszutauschen sind.

Die Aufgabe der vorliegenden Erfindung bestand darin, ein Verfahren zur Bildung von Biureten aus Isocyanaten zu erarbeiten, mit dem Wasser oder Wasserdampf lediglich während der Reaktion anwesend ist und nicht in nachgeschalteten Anlagenteilen auftritt.

Die Aufgabe wurde gelöst durch ein Verfahren zur Herstellung von biuretgruppenhaltigen Polyisocyanaten aus
a) mindestens einem Di- und/oder Polyisocyanat,
b) Wasser und/oder Wasserdampf (Wasser(dampf)) als Biuretisierungsmittel,
c) sowie gegebenenfalls mindestens einem Katalysator unter Freisetzung von Kohlenstoffdioxid, umfassend
   i) Vermischen der Komponenten a) und b) sowie gegebenenfalls c) in einer Mischeinrichtung mit einer Mischenergie von mindestens 0,05 × 10⁶ J/kg Wasser(dampf) und
   ii) Leiten des aus i) erhaltenen Reaktionsgemisches in mindestens einen Reaktor, in dem das Reaktionsgemisch, Wasser(dampf) und Kohlenstoffdioxid im Gleichstrom oder Gegenstrom, bevorzugt im Gleichstrom geführt werden.

Der Vorteil der vorliegenden Erfindung besteht darin, daß in dem erfindungsgemäß hergestellten Produkt nach Verlassen der Reaktionszone keine signifikante Biuretisierung mehr auftritt, die zu Verstopfung in Bauteilen führen könnte. Zudem sind die Bauteile, die zur Durchführung des erfindungsgemäßen Verfahrens eingebaut werden, so ausführbar, daß sie leicht zu warten und/oder auszutauschen sind.

Wasser und/oder Wasserdampf wird in dieser Schrift begrifflich kurz als Wasser(dampf) zusammengefaßt.

Als Di- und Polyisocyanate a) für das erfindungsgemäße Verfahren sind besonders geeignet (cyclo)aliphatische Isocyanate, also solche Verbindungen, die mindestens 2, bevorzugt 2 bis 6, besonders bevorzugt 2 bis 4, ganz besonders bevorzugt 2 bis 3 und insbesondere 2 Isocyanatgruppen aufweisen, die an Kohlenstoffatome gebunden sind, die Teil eines aliphatischen und/oder cyclischen Systems sind.

Geeignete Diisocyanate sind bevorzugt Diisocyanate mit 4 bis 20 C-Atomen.

Aromatische Isocyanate sind solche, die mindestens ein aromatisches Ringsystem enthalten.

Cycloaliphatische Isocyanate sind solche, die mindestens ein cycloaliphatisches Ringsystem enthalten.

Aliphatische Isocyanate sind solche, die ausschließlich gerade oder verzweigte Ketten enthalten, also acyclischen Verbindungen.

Besonders bevorzugt sind als aliphatische Diisocyanate Tetramethylendiisocyanat, Hexamethylendiisocyanat (1,6-Diisocyanatohexan), Octamethylendiisocyanat, Decamethylendiisocyanat, Dodecamethylendiisocyanat, Tetradecamethylendiisocyanat, Derivate des Lysindiisocyanates, Tetramethylxylylendiisocyanat, 2,4,4- und/oder 2,2,4-Trimethylhexandiisocyanat oder Tetramethylhexandiisocyanat, und als cycloaliphatische Diisocyanate 1,4-, 1,3- oder 1,2-Diisocyanatocyclohexan, 4,4'- oder 2,4'-Di(isocyanatocyclohexyl)methan, 1-Isocyanato-3,3,5- trimethyl-5-(isocyanatomethyl)-cyclohexan (Isophorondiisocyanat), 1,3- oder 1,4-Bis(isocyanatomethyl)cyclohexan oder 2,4-, oder 2,6-Diisocyanato-1-methylcyclohexan. Denkbar ist auch die Durchführung mit aromatischen Isocyanaten, besonders 2,4- und/oder 2,6-Toluylendiisocyanat (TDI), 2,4'- und/oder 4,4'- Diisocyanatodiphenylmethan (MDI) oder deren Gemische. Bevorzugt handelt es sich um aliphatische oder cycloaliphatische Isocyanate, besonders bevorzugt um Hexamethylendiisocyanat oder Isophorondiisocyanat. Es können auch Gemische der genannten Diisocyanate vorliegen.

2,2,4- und 2,4,4-Trimethyl-1,6-hexamethylendiisocyanat fallen dabei produktionsbedingt zumeist als Isomerengemisch im Verhältnis 1,5:1 bis 1:1,5, bevorzugt 1,2:1 - 1:1,2, besonders bevorzugt 1,1:1-1:1,1 und ganz besonders bevorzugt 1:1 an.

Diisocyanate können industriell z.B. durch Phosgenierung von Diaminen nach den in DE-PS 20 05 309 und DE-OS 2 404 773 beschriebenen Verfahren oder durch ein phosgenfreies Verfahren (Spaltung von Biurethanen) wie beschrieben in der EP-B-0 126 299 (US-A-4 596 678), EP-B-0 126 300 (US-A-4 596 679), EP-A-0 355 443 (US-A-5 087 739) sowie in der EP-A-0 568 782 beschrieb hergestellt werden. Erfindungsgemäß spielt es keine Rolle, ob das eingesetzte Isocyanat nach einem phosgenfreien oder einem phosgenhaltigen Herstellungsweg erhalten worden ist.

Isocyanate, die aus einem Phosgenierungsprozeß stammen, weisen häufig einen Gesamtchlorgehalt von 100-400 mg/kg auf, wohingegen die erfindungsgemäß bevorzugten Isocyanate, einen Gesamtchlorgehalt von weniger als 80 mg/kg aufweisen, bevorzugt weniger als 60, besonders bevorzugt weniger als 40, ganz besonders bevorzugt weniger als 20 und insbesondere weniger als 10 mg/kg. Der Gesamtbromgehalt beträgt in der Regel weniger als 15 mg/kg, bevorzugt weniger als 10 mg/kg und speziell weniger als 5 mg/kg. Speziell werden solche Isocyanate eingesetzt, die aus einem phosgenfreien Prozeß stammen.

Man kann noch zwischen hydrolysierbarem und nicht-hydrolysierbarem Halogengehalt unterscheiden, wobei der hydrolysierbare Anteil in der Regel ca. 0,5 - 80%, bevorzugt 1 bis 50% und besonders bevorzugt 2 bis 30% des Gesamthalogengehalts ausmacht.

Der Gehalt an hydrolysierbarem Chlor wird bestimmt gemäß der ASTM D4663-98 und beträgt bevorzugt weniger als 40 ppm, besonders bevorzugt weniger als 30 ppm und ganz besonders bevorzugt weniger als 20 Gew.ppm.

Die biuretgruppenhaltigen Polyisocyanate werden durch Einmischung eines Stromes des Biuretisierungsmittels Wasser(dampf) in einen isocyanathaltigen Strom und thermische Behandlung in mindestens einem Reaktor hergestellt. Dabei reagieren die Isocyanatgruppen mit Wasser(dampf) unter Ausbildung von Biuretgruppen, wobei Kohlenstoffdioxid (CO₂) freigesetzt wird, das sich teilweise im Reaktionsgemisch löst und/oder teilweise eine Gasphase neben dem Reaktionsgemisch ausbilden kann.

Durch die Anwesenheit des Kohlenstoffdioxids im Reaktionsgemisch können sich, je nach verwendetem Katalysator (siehe unten), oxadiazintriongruppenhaltige Polyisocyanate bilden. In der Regel beträgt der Anteil an oxadiazintriongruppenhaltigen Polyisocyanaten im Reaktionsgemisch weniger als 1 Gew%, bevorzugt 0,75 Gew%, besonders bevorzugt weniger als 0,5 Gew%, ganz besonders bevorzugt weniger als 0,3 Gew% und insbesondere weniger als 0,1 Gew%.

Die Reaktion wird bevorzugt in Gegenwart mindestens eines Katalysators c) durchgeführt.

Dabei kann es sich beispielsweise um OH-acide Verbindungen handeln, wie bekannt aus der DE-A1 44 43 885. Diese haben den Vorteil, daß sie schwerflüchtig sind und deshalb, gegebenenfalls als Salze, aus dem Produktgemisch abfiltriert werden können oder als nicht störende Verbindungen im Endprodukt verbleiben und während der Reaktion ebenfalls nicht störende Zersetzungs- oder Nebenprodukte bilden. Ein weiterer Vorteil ist die gute katalytische Aktivität der Säuren.

Als OH-acide Verbindungen kommen für das erfindungsgemäße Verfahren insbesondere Protonensäuren in Betracht. Bevorzugt können eingesetzt werden und haben sich besonders bewährt: Phosphorsäuren und/oder deren Mono- und/oder Dialkylester oder -arylester und/oder Hydrogensulfate. Verwendung finden vorzugsweise Mono- und/oder Dialkylester oder -arylester der Phosphorsäure, deren aliphatische, verzweigt aliphatische, araliphatische oder aromatische Reste 1 bis 30, vorzugsweise 4 bis 20, Kohlenstoffatome aufweisen. Bevorzugt kommen Di-(2-ethylhexyl)phosphat, Di-(n-butyl)phosphat und Di-hexadecylphosphat zur Anwendung.

Insbesondere in Frage kommen Dibutylphosphat und Di-iso-propylphosphat. Bevorzugt wird Di-(2-ethylhexyl)-phosphat.

Als Protonensäuren kommen beispielsweise Hydrogensulfate in Betracht, insbesondere Tetralkylammoniumhydrogensulfate in Betracht, deren aliphatische, verzweigt aliphatische oder araliphatische Reste 1 bis 30, vorzugsweise 4 bis 20, Kohlenstoffatome aufweisen.

Weitere Beispiele sind Sulfonsäuren, wie z.B. Methansulfonsäure, Ethansulfonsäure, Propansulfonsäure, 2- bzw. 4-Toluolsulfonsäure, Benzolsulfonsäure, Cyclododecansulfonsäure, Camphersulfonsäure oder Naphthalin-1- oder -2-sulfonsäure, oder auch Mono- und Dicarbonsäuren, wie z.B. Ameisensäure, Essigsäure, Propionsäure, Buttersäure, Pivalinsäure, Stearinsäure, Cyclohexancarbonsäure, Oxalsäure, Malonsäure, Bersteinsäure, Adipinsäure, Benzoesäure oder Phthalsäure.

Die beispielsweise in EP-A-259 233 beschriebenen (ar)aliphatischen Carbonsäuren erweisen sich als weniger wirksam.

Besonders bevorzugt sind Protonensäuren mit einem pK_{S}-Wert < 10. Bevorzugte saure Katalysatoren sind Phosphorsäure bzw. die oben erwähnten Phosphorsäureester, wie z. B. Methylphosphat, Ethylphosphat, n-Butylphosphat, n-Hexylphosphat, 2-Ethylhexylphosphat, Isooctylphosphat, n-Dodecylphosphat, Dimethylphosphat, Diethylphosphat, Di-n-propylphosphat, Di-n-butylphosphat, Di-n-amylphosphat, Diisoamylphosphat, Di-n-decylphosphat, Diphenylphosphat oder Dibenzylphosphat sowie Gemische daraus.

Besonders bevorzugt sind Dialkylphosphate der genannten Art. Ganz besonders bevorzugte Katalysatoren sind Di-n-butylphosphat und Di-(2-ethylhexyl) phosphat.

Diese Säuren kommen beim erfindungsgemäßen Verfahren in Mengen von 0,01 bis 1,0 Gew.-%, vorzugsweisevon 0,02 bis 0,5 Gew.-% und ganz besonders bevorzugt von 0,05 bis 0,5 Gew.-%, bezogen auf die Gesamtmenge an eingesetzten Diisocyanaten. Die Säuren können gelöst oder dispergiert in einem geeigneten Lösungsmittel zugegeben werden. Vorzugsweise werden die Säuren in Substanz zugegeben.

Die genannten OH-aciden Verbindungen haben den Vorteil, daß sie häufig schwerflüchtig sind und deshalb, gegebenenfalls als Salze, aus dem Produktgemisch abfiltriert werden können oder als nicht störende Verbindungen im Endprodukt verbleiben oder während der Reaktion ebenfalls nicht störende Zersetzungs- oder Nebenprodukte bilden. Ein weiterer Vorteil ist die gute katalytische Aktivität der Säuren.

Als Katalysator können ferner beispielsweise starke anorganische Lewis- oder Brønstedt-Säuren, wie beispielsweise Bortrifluorid, Aluminiumtrichlorid, Schwefelsäure, Phosphorige Säure, Salzsäure und/oder Salze aus stickstoffhaltigen Basen und anorganischen und/oder organischen Säuren, wie sie beschrieben sind in DE-A-19 31 055, Seite 3, letzter Absatz bis Seite 6, erster ganzer Absatz, was hiermit durch Referenznahme Gegenstand der vorliegenden Offenbarung sei, eingesetzt werden.

Falls gewünscht, kann zusätzlich noch eine kleine Menge eines Stabilisators zugesetzt werden ausgewählt aus der Gruppe Harnstoff, Ammoniak, Biuret, Harnstoffderivaten oder Carbonsäureamiden, wie sie beschrieben sind in WO 96/25444, bevorzugt Harnstoff, N-Methylharnstoff, N-Ethylharnstoff, N,N-Dimethylharnstoff, N,N'-Dimethylharnstoff, N,N-Diethylharnstoff, N,N'-Diethylharnstoff, Ethylenharnstoff oder Phenylharnstoff, besonders bevorzugt ist Harnstoff.

Derartige Stabilisatoren werden in Mengen von 0,01 - 2,0, bevorzugt 0,05 - 1 mol%, bezogen auf die Isocyanatgruppen in (a) eingesetzt.

In einer bevorzugten Ausführungsform werden dieses Stabilisatoren in mindestens einem Lösungsmittel gelöst oder dispergiert, wie sie nachfolgend aufgeführt sind, besonders bevorzugt in Wasser.

Um die Bildung von unlöslichen Polyharnstoffen noch besser zu unterdrücken, können gegebenenfalls zusätzlich Lösungsmittel als Lösungsvermittler verwendet werden. Hierfür geeignet sind beispielsweise Dioxan, Tetrahydrofuran, Alkoxyalkylcarboxylate wie z.B. Triethylenglykoldiacetat, Butylacetat, Ethylacetat, 1-Methoxypropyl-2-acetat, Propylenglykoldiacetat, 2-Butanon, 4-Methyl-2-pentanon, Cyclohexanon, Hexan, Toluol, Xylol, Benzol, Chlorbenzol, o-Dichlorbenzol, Kohlenwasserstoffgemische, Methylenchlorid und/oder Trialkylphosphate.

Beispiele für Kohlenwasserstoffe als Lösungsmittel sind aromatische und/oder (cyclo)aliphatische Kohlenwasserstoffe und deren Gemische, halogenierte Kohlenwasserstoffe, Ester und Ether.

Besonders bevorzugt sind ein- oder mehrfach alkylierte Benzole und Naphthaline sowie deren Gemische.

Als aromatische Kohlenwasserstoffgemische sind solche bevorzugt, die überwiegend aromatische C₇- bis C₁₄-Kohlenwasserstoffe umfassen und einen Siedebereich von 110 bis 300 °C umfassen können, besonders bevorzugt sind Toluol, o-, m- oder p-Xylol, Trimethylbenzolisomere, Tetramethylbenzolisomere, Ethylbenzol, Cumol, Tetrahydronaphthalin und solche enthaltende Gemische.

Beispiele dafür sind die Solvesso®-Marken der Firma ExxonMobil Chemical, besonders Solvesso® 100 (CAS-Nr. 64742-95-6, überwiegend C₉ und C₁₀-Aromaten, Siedebereich etwa 154 - 178 °C), 150 (Siedebereich etwa 182 - 207 °C) und 200 (CAS-Nr. 64742-94-5), sowie die Shellsol®-Marken der Firma Shell. Kohlenwasserstoffgemische aus Paraffinen, Cycloparaffinen und Aromaten sind auch unter den Bezeichnungen Kristallöl (beispielsweise Kristallöl 30, Siedebereich etwa 158 - 198 °C oder Kristallöl 60: CAS-Nr. 64742-82-1), Testbenzin (beispielsweise ebenfalls CAS-Nr. 64742-82-1) oder Solventnaphtha (leicht: Siedebereich etwa 155 - 180 °C, schwer: Siedebereich etwa 225 - 300 °C,) im Handel erhältlich. Der Aromatengehalt derartiger Kohlenwasserstoffgemische beträgt in der Regel mehr als 90 Gew%, bevorzugt mehr als 95, besonders bevorzugt mehr als 98 und ganz besonders bevorzugt mehr als 99 Gew%. Es kann sinnvoll sein, Kohlenwasserstoffgemische mit einem besonders verringerten Gehalt an Naphthalin einzusetzen.

Erfindungsgemäß bevorzugt eingesetzt werden Methoxypropylacetat, Trimethylphosphat, Tri-n-butylphosphat und Triethylphosphat oder beliebige Mischungen dieser Verbindungen.

Die erfindungsgemäße Reaktion wird jedoch bevorzugt in Abwesenheit von Lösungsmitteln durchgeführt.

Bei dem Biuretisierungsmittel b) handelt es sich im erfindungsgemäßen Verfahren um Wasser und/oder Wasserdampf.

Der Wasser(dampf)strom kann auch noch zusätzliche Inertströme enthalten, z.B. einen flüssigen oder gasförmigen Inertstrom enthalten. Bevorzugt wird der Inertstrom gasförmig zugegeben. Als Inertmedium kommen alle Gase in Frage, die nicht wesentlich, d.h. bei den Reaktionsbedingungen zu weniger als 5 mol%, bevorzugt zu weniger als 2 mol%, besonders bevorzugt zu weniger als 1 mol% mit dem Isocyanatstrom, dem wasser(dampf)haltigen Strom und/oder dem Katalysator reagieren. Beispiele dafür sind CO₂, CO, N₂, He, Ar, Kohlenwasserstoffe, wie Methan etc., sowie deren Mischungen. Bevorzugt werden Kohlendioxid und/oder Stickstoff eingesetzt. Besonders bevorzugt wird Stickstoff eingesetzt.
Denkbar sind auch Stickstoff-Sauerstoff- oder bevorzugt Stickstoff-Luft-Gemische mit einem Sauerstoffgehalt von weniger als 10 Vol%, bevorzugt weniger als 8 Vol% und besonders bevorzugt von ca. 6 Vol%.

Das Molenverhältnis von Inertstrom zu Wasser(dampf)strom beträgt von 1 zu 1000 bis 1 zu 0,1, besonders bevorzugt von 1 zu 100 bis 1 zu 1, ganz besonders bevorzugt von 1 zu 10 bis 1 zu 1 und insbesondere von 1 zu 5 bis 1 zu 1.

Als Mischeinrichtung in Stufe i) können statische oder bewegte Mischeinrichtungen verwendet werden.

Beispiele dafür sind Pumpen, Mischpumpen, Düsenmischeinrichtungen oder Begasungsrührer, sowie Kombinationen davon. Bevorzugt werden Düsenmischeinrichtungen verwendet.

Die Vermischung findet in der Regel bei Temperaturen von mindestens 30 °C, bevorzugt von mindestens 50 °C, besonders bevorzugt von mindestens 80 und ganz besonders bevorzugt von mindestens 100 °C statt.

Die obere Grenze der Temperatur während der Vermischung liegt in der Regel bei 250 °C, bevorzugt bei 190 °C, besonders bevorzugt bei 150°C.

Bevorzugt beträgt die Mischzeit in der Mischeinrichtung nicht mehr als ein Zehntel der gesamten mittleren Verweilzeit, also der mittleren Zeit zwischen Starten und Stoppen der Reaktion, besonders bevorzugt nicht mehr als ein Zwanzigstel, ganz besonders bevorzugt nicht mehr als ein Dreißigstel und insbesondere nicht mehr als ein Hundertstel.

Die Mischzeit in dieser Mischeinrichtung beträgt üblicherweise von 0,01 s bis 120 s, bevorzugt von 0,05 bis 60 s, besonders bevorzugt von 0,1 bis 30 s, ganz besonders bevorzugt von 0,5 bis 15 s und insbesondere von 0,7 bis 5 s. Als Mischzeit ist diejenige Zeit zu verstehen, die von dem Beginn des Mischvorgangs vergeht, bis 97,5 % der Fluidelemente des erhaltenen Gemisches einen Mischungsbruch haben, der bezogen auf den Wert des theoretischen Endwert des Mischungsbruchs des erhaltenen Gemisches beim Erreichen des Zustandes perfekter Mischung weniger als 2,5 % von diesem Endwert des Mischungbruches abweichen. (zum Konzept des Mischungsbruches siehe z.B. J.Warnatz, U.Maas, R.W. Dibble: Verbrennung, Springer Verlag, Berlin Heidelberg New York, 1997, 2. Auflage, S. 134.)

Der Absolutdruck am Ausgang der Mischeinrichtung liegt im Bereich von 0,3 bar bis 10 bar, bevorzugt von 0,6 bar bis 7 bar, besonders bevorzugt von 0,8 bar bis 5 bar.

Bei Verwendung einer statischen Mischeinrichtung liegt der Vordruck auf der Isocyanat-Zufuhrseite zur Mischeinrichtung 2 bis 100 bar, bevorzugt 4 bis 60 bar, besonders bevorzugt 10 bis 50 bar höher als der Druck auf der Ausgangsseite der Mischeinrichtung.

Der Vordruck auf der Wasser(dampf)zufuhrseite zur Mischeinrichtung bei Verwendung einer statischen Mischeinrichtung bei 0,2 bis 20 bar, bevorzugt, 0,4 bis 10 bar, besonders bevorzugt 1 bis 5 bar höher als auf der Ausgangsseite der Mischeinrichtung.

Dabei ist unabhängig von der Art der Mischeinrichtung pro kg eingemischtem Wasser(dampf) eine Mischarbeit von 0,05 × 10⁶ bis 20 ×10⁶, bevorzugt von 0,2 × 10⁶ bis 10 × 10⁶, besonders bevorzugt von 0,5 × 10⁶ bis 7 × 10⁶ Joule notwendig, um das Wasser oder den Wasserdampf fein einzumischen.

In der Mischeinrichtung werden der Diisocyanat enthaltende Strom und der Wasser(dampf)strom mit oder ohne Inertgas vermischt.

Als Mischdüsen können nicht-axialsymmetrische oder bevorzugt axialsymmetrische Mischdüsen verwendet werden. Bei Verwendung von axialsymmetrischen Mischdüsen wird üblicherweise einer der Eduktströme über ein koaxiales Einleitrohr oder mehrere koaxial angeordnete Einleitrohre in ein Mischrohr eingedüst. Der zweite Eduktstrom wird über den Ringspalt zwischen dem koaxialen Einleitrohr und dem Mischrohr eingedüst. Das Durchmesserverhältnis des koaxialen Einleitrohrs zum Mischrohr beträgt üblicherweise 0,05 bis 0,95, bevorzugt 0,2 bis 0,8. Der wasser(dampf)haltige Strom kann zentral über die innere koaxiale Einleitung zugedüst werden oder über den Ringspalt. Bevorzugt wird der Wasser(dampf)strom über die innere koaxiale Eindüsung eingebracht.

Die Länge des Mischrohres nach der Düse beträgt üblicherweise das 2 bis 20-fache des Mischrohrdurchmessers, bevorzugt das 4 bis 10 -fache des Mischrohrdurchmessers.

Die Geschwindigkeiten im Mischrohr betragen üblicherweise 1 bis 100 m/s, bevorzugt 5 bis 30 m/s.

Bei der Verwendung von nicht axialsymmetrischen Mischdüseneinrichtungen können Mischkammerkonstruktionen verwendet werden, bei denen die Eduktströme in eine Mischkammer eingeleitet werden und der Gemischstrom der Mischkammer entnommen wird. Weiter mögliche nicht axialsymmetrische Mischdüseneinrichtungen sind T- oder Y-Mischer.

Als statische Mischer können alle üblichen statischen Mischer (z.B. Sulzer SMX/SMV) oder auch Düsen- oder Blendenmischeinrichtungen verwendet werden.

Die Wasser(dampf)stromgeschwindigkeit in der Eindüsung variiert zwischen 10 bis 400 m/s, bevorzugt zwischen 30 bis 350 m/s besonders bevorzugt zwischen 60 und 300 m/s. Die Isocyanatstromgeschwindigkeiten variieren in der Eindüsung zwischen 5 bis 100 m/s, bevorzugt zwischen 20 bis 80 m/s, besonders bevorzugt zwischen 30 bis 70 m/s.

Wasser(dampf) wird im allgemeinen in Mengen von 0,5 bis 20 mol-%, bevorzugt 2 bis 15 mol-%, bezogen auf die Isocyanatgruppen im frisch zugeführten Isocyanat (a), eingesetzt.

Als Reaktionsapparatur können alle üblichen volumenaufweisenden Reaktoren, wie Rührkessel, Strahlschlaufenreaktoren, Blasensäulen, Rohrreaktoren, Behälter, Kolonnen eingesetzt werden. Es sind auch Kombinationen oder mehrfache Verwendung der Apparatearten möglich. Z.B. kann ein Rührkessel mit einem Rohrreaktor kombiniert werden. Der Prozess kann auch in einer Kaskade von Rührkesseln durchgeführt werden.

Mit Vorteil ist die Strömung in der Reaktionsaparatur zumindest teilweise, bevorzugt vollständig eine turbulente.

Wenn es sich bei der Reaktionsapparatur um einen oder mehrere Rührkessel handelt, so wird der Strömungszustand bevorzugt so eingestellt, dass die den Leistungseintrag kennzeichnende Newton-Zahl nicht umgekehrt proportional von der mit dem Rührerdurchmesser gebildeten Reynolds-Zahl bei Variation der Drehzahl abhängt. Besonders bevorzugt wird der Strömungszustand so eingestellt, dass die Newton-Zahl nicht von der Reynolds-Zahl bei Variation der Drehzahl abhängt.

Wenn es sich um einen Rohrreaktor ohne Einbauten handelt, so ist die Reynolds-Zahl bevorzugt mindestens 2300, besonders bevorzugt mindestens 2700, ganz bevorzugt mindestens 3000, insbesondere mindestens 4000, mindestens 7000 oder speziell mindestens 10000.

Bevorzugt wird mindestens ein längs durchströmter Rührkessel mit einem Durchmesser zu Längenverhältnis von 1 zu 1,2 bis 1 zu 10, bevorzugt von 1 zu 1,5 bis 1 zu 6 verwendet. In einer weiteren besonders bevorzugten Ausführung wird dieser Rührkessel durch Einbauten kaskadiert. Als Einbauten sind Lochbleche, Siebe, Schlitzböden, konzentrische Scheiben möglich, wie es beschrieben ist in der DE-A1 195 25 474. Durch die Einbauten wird der Rührkessel in bevorzugt 2 bis 10 Segmente unterteilt, besonders bevorzugt in 3 bis 6 Segmente, die von anderen durch die genannten Einbauten getrennt werden. Selbstverständlich kann es sich auch um voneinander getrennte Rührkessel handeln.

Der volumenspezifische Leistungseintrag in diesem Rührkessel sollte bei mindestens 0,1 Watt/I, bevorzugt mindestens 0,3, besonders bevorzugt mindestens 0,5 Watt/l liegen. In der Regel sind bis 20 Watt/I, bevorzugt bis 6 Watt/I und besonders bevorzugt bis 2 Watt/I ausreichend.

Die Leistung kann über alle möglichen Rührertypen, wie Schrägblatt, Anker, Scheiben, Turbinen, Balken-Rührer eingetragen werden. Bevorzugt werden Scheiben- und Turbinen-Rührer eingesetzt.

Es können auch mehrere Rührer auf der Welle installiert sein. Bevorzugt wird pro Segment der Kaskade ein Rührer auf der Welle verwendet. Der Durchmesser der Rührelemente beträgt das 0,1 bis 0,9 fache des Rührkesseldurchmessers, bevorzugt das 0,2 bis 0,6 fache des Rührkesseldurchmessers.

Der Rührkessel oder kaskadierte Rührkessel kann mit oder ohne Stromstörer betrieben werden. Bevorzugt erfolgt der Betrieb mit Stromstörern. Der Betrieb erfolgt üblicherweise mit 1 bis 10 Stromstörern, bevorzugt mit 2 bis 4 Stromstörem je Segment.

Das Wasser(dampf)/Isocyanat-Gemisch wird dem Reaktionsapparat zugeführt. In der bevorzugten Ausführung, dass es sich bei dem Reaktionsapparat um einen überwiegend senkrecht angeordneten Apparat handelt (beispielsweise senkrechter Rohrreaktor, Kolonne oder schlanker Rührkessel) kann die Zufuhr des Wasser(dampf)-Isocyanatgemisches von unten erfolgen (Gleichstrom der flüssigen Phase mit dem CO₂) oder von oben (Gegenstrom zu CO₂), bevorzugt von unten.

Die Entnahme des sich bei der Vermischung oder im Reaktor bildenden CO₂ kann an jeder Stelle im System erfolgen. Bevorzugt erfolgt die Entnahme erst nach vollständiger Umsetzung des Wasser(dampf)s.

Die Verweilzeit im Reaktor ii) liegt im Bereich von 0,2 bis 10 Stunden, bevorzugt von 0,4 bis 6 Stunden und besonders bevorzugt 0,5 bis 3 Stunden. Die Reaktionszeit wird dabei günstigerweise so gewählt, daß am Ende der theoretische NCO-Wert erreicht ist. Der theoretische NCO-Wert ist der NCO-Wert, den die Reaktionsmischung aufweist, wenn die gesamte eingesetzte Menge an Wasser(dampf) die theoretisch zu erwartende Menge an Biuretgruppen gebildet hat.

Die Temperatur beträgt im Bereich der Reaktionsstrecke im Bereich von 30 bis 250 °C, bevorzugt von 100 bis 190 °C, besonders bevorzugt im Bereich von 120 bis 170°C.

Es kann sinnvoll sein, die Temperatur von Segment zu Segment zu ändern, beispielsweise kann die Temperatur im Verlauf des Reaktors ansteigen, fallen oder gleich bleiben, bevorzugt steigt sie oder bleibt gleich.

Der Absolutdruck im Reaktor liegt im Bereich von 0,3 bis 10 bar, bevorzugt von 0,6 bis 4 bar, besonders bevorzugt von 0,8 bis 2 bar.

Bevorzugt wird dem Reaktionssystem noch ein Katalysator c) beigefügt, so kann das Einmischen des Katalysatorstroms separat im Reaktor oder auch an mehreren Stellen erfolgen oder auch zu einem der Ströme, die der Mischdüse zugeführt werden. Bevorzugt wird der Katalysator in einen der Ströme eingemischt, die zur Mischdüse geführt werden. Besonders bevorzugt wird der Katalysatorstrom dem Isocyanatgruppen enthaltenden Strom zugeführt, der in die Mischeinrichtung geht.

In der einfachsten Ausführung besteht die Erfindung aus der Kombination Mischeinrichtung i) und Reaktor ii). Hier wird dann der Isocyanatstrom mit oder ohne Katalysator c) mit dem wasser(dampf)haltigen Strom b) vermischt und dann in den Reaktor ii) eingebracht. Dabei müssen die Mischeinrichtung i) und der Reaktor ii) nicht apparatetechnisch getrennt sein, sondern der Reaktor kann sich auch direkt an die Mischeinrichtung anschließen.

In der Mischeinrichtung ii) kann die Reaktion bereits unmittelbar nach Vermischung der Komponenten beginnen, so daß die Reaktion nicht notwendigerweise auf den Reaktor beschränkt ist.

Eine weitere Ausführung der Erfindung besteht im Aufbau eines Mischkreises, enthaltend eine Pumpe zum Umpumpen der Isocyanat/Polyisocyanatmischung, gegebenenfalls versehen mit Pumpenvorlage und/oder Mischeinrichtung.

Unter einem Mischkreis wird dabei ein Umpumpkreislauf verstanden, der mindestens eine Pumpe und mindestens eine Mischeinrichtung, beispielsweise statische Mischer und/oder Mischelemente, enthält und in den mindestens eine der zu vermischenden Komponenten dosiert wird, bevorzugt vor der Pumpe. Der Umpumpkreislauf kann darüberhinaus eine Pumpenvorlage enthalten. Der Umpumpkreislauf kann weiterhin mindestens einen Wärmetauscher enthalten.

Dabei saugt die Pumpe aus der Pumpenvorlage den isocyanathaltigen Strom und fördert diesen zur Mischeinrichtung von wo aus dieser wieder zur Pumpenvorlage geführt wird. Aus diesem Kreis, bevorzugt zwischen Mischeinrichtung und Vorlage, kann ein Teil dem Kreislaufstrom entnommen und in den Reaktor ii) geleitet werden.

Alternativ oder zusätzlich können auch frisches Isocyanat und Katalysator in den Reaktor geführt werden, bevorzugt an den Reaktoranfang, besonders bevorzugt in die ersten 25 % der Reaktorstrecke, ganz besonders bevorzugt in die ersten 10%. Die Vermischung findet dann innerhalb des Reaktors statt und gegebenenfalls zusätzlich dadurch, daß ein Teilstrom dem Reaktor an beliebiger Stelle entnommen und in den Mischkreis eingespeist wird.

In einer bevorzugten Ausführungsform kann Katalysator auch an mehreren Stellen des Reaktors und/oder Mischkreises eingespeist werden.

Bevor der Strom in die Mischeinrichtung eintritt, wird dem isocyanathaltigen Strom der Frischisocyanatstrom beigefügt. Zur Unterstützung der Einmischung des Frischisocyanatstroms in den isocyanathaltigen Umpumpstrom kann eine statische oder eine sonstige Mischeinrichtung verwendet werden.

In der Mischeinrichtung werden dann der isocyanathaltige Strom und der wasser-(dampf)haltige Strom vermischt. Der Austrag aus der Mischeinrichtung wird bei der Ausführung als Mischkreis zumindest teilweise wieder in die Pumpenvorlage befördert. Der restliche Teilstrom des Austrags wird in den Reaktor überführt. Dabei ist die Verwendung der Pumpenvorlage nicht zwingend, sie erleichtert nur den regelungstechnisch sinnvollen Betrieb der Pumpe.

Eine bevorzugte Ausführung der Erfindung besteht in der Kombination eines Mischkreises mit dem Reaktor. Dazu wird dem Reaktor ein isocyanathaltiger Strom über eine Pumpe entnommen und zur Mischeinrichtung befördert. In der Mischeinrichtung wird der wasser(dampf)haltige Strom eingemischt. Der aus der Mischeinrichtung austretende Strom wird in den Reaktor zurückgeführt. Der Katalysatorstrom und der Frischisocyanatstrom werden entweder dem isocyanathaltigen Strom vor der Mischdüse zugeführt und/oder direkt in den Reaktor eingeleitet.

In einer besonders bevorzugten Ausführung besteht der Reaktor aus einem kaskadierten Rührkessel. Die Isocyanatlösung, die zur Mischeinrichtung gefördert wird, wird dem untersten Segment des kaskadierten Rührkessels entnommen.

Gemäß einer besonders vorteilhaften Ausbildung des erfindungsgemäßen Verfahrens wird die Verteilung des gasförmigen Anteils im flüssigen Reaktanden durch in den Rührreaktor eingebaute Störelemente verbessert. Als Trennelemente zwischen den einzelnen Segmenten können mit Abstand voneinander angeordnete, mit zentralen Öffnungen versehene Scheiben verwendet werden. Zusätzlich können in Längsrichtung des Rührreaktors verlaufende Störleisten eingesetzt werden. Dem als Biuretisierungmittel zugeführten Reaktanden, vorzugsweise Wasserdampf, kann 10 bis 95 Vol.% Stickstoff und/oder Kohlendioxid beigemischt werden. Für die Reaktion wird eine Temperatur von 60 bis 200 °C, vorzugsweise 100 bis 150 °C, eingestellt. Vorzugsweise wird das am Kopfende des Rührreaktors abströmende Abgas mit Diisocyanat gewaschen, das auf eine Temperatur von ca. 0 bis 20 °C vorgekühlt sein kann, aber nicht zwangsläufig sein muß. Das aus dem Waschschritt ausgetragene Diisocyanat wird anschließend dem Verfahren zugeführt. Eine derartige Wäsche des Abgases mit frischem Isocyanat verringert Ablagerungen von Polyharnstoffrückständen im Abgassystem.

Zur Durchführung des Verfahrens ist erfindungsgemaß ein Rührreaktor vorgesehen, der einen senkrecht stehenden rohrförmigen Behälter aufweist, in dem parallel zur Längsachse eine Antriebachse drehbar befestigt ist, an der mindestens zwei Scheibenrührer mit Abstand voneinander befestigt sind und bei dem zwischen diesen Scheibenrührern an der Innenwand des Behälters befestigte Trennelemente angeordnet sind, die eine zentrale Öffnung aufweisen. Es hat sich herausgestellt, daß in Abhängigkeit von den Reaktionsparametern zweckmäßigerweise 2 bis 7 Scheibenrührer und 1 bis 6 Trennelemente eingesetzt werden. Das Verhältnis der Öffnung der Trennelemente zu deren Gesamtfläche ergibt sich aus der Rührergröße.

Gemäß einer Weiterbildung des erfindungsgemäßen Rührreaktors können an dessen Innenwand parallel zu seiner Längsachse verlaufende, radial sich nach innen erstreckende Stromstörleisten angeordnet sein. Diese können vorzugsweise mit Abstand zur Innenwand des Reaktors befestigt sein. Vorteilhafterweise werden mindestens 2, bevorzugt 2 - 6, besonders bevorzugt 2 bis 4 und ganz besonders bevorzugt 2, 3 oder 4 im gleichen Winkelabstand voneinander angeordnete Störleisten vorgesehen. Die Stromstörleisten können in ihrer Breite dabei das 0,1- 0,4-fache des Durchmesser des Behälters einnehmen.

Ferner kann es sinnvoll sein, in dem Reaktor ungerührte Segmente vorzusehen (Beruhigungszonen). In einer bevorzugten Ausführungsform der Erfindung können sich am Ein- und/oder Ausgang, besonders bevorzugt befinden sich am Ein- und Ausgang derartige Beruhigungszonen.

Der Rührreaktor kann von einem beheizbaren Mantel oder aufgeschweißten Voll- oder Halbrohren umschlossen sein. Vorteilhafterweise ist das Kopfende des Rührreaktors mit einem Behälter (Abgaswäscher) verbunden, in dessen unterem Teil ein Kühler eingebaut sein kann. Oberhalb dieses Kühlers befindet sich ein Injektor für den flüssigen Reaktanden. Diese Einheit mündet in einer Abgasleitung.

Vorteilhafterweise liegt das Verhältnis der Höhe des Rührreaktors zu dessen Durchmesser im Bereich 2 bis 6 und ist vorzugsweise größer als 4,5.

Mit Reaktoren der vorbeschriebenen Bauweise läßt sich das erfindungsgemäße Verfahren besonders vorteilhaft durchführen.

Weitere Einzelheiten und Vorteile der Erfindung können der Beschreibung der in der Zeichnung dargestellten Versuchsanlage entnommen werden. Es zeigen:
Fig. 1 eine erfindungsgemäße Anlage mit kaskadenförmigem Rührreaktor,
Fig. 2 eine Draufsicht auf einen im Rührreaktor angeordneten Scheiben- und Schrägblattrührer.

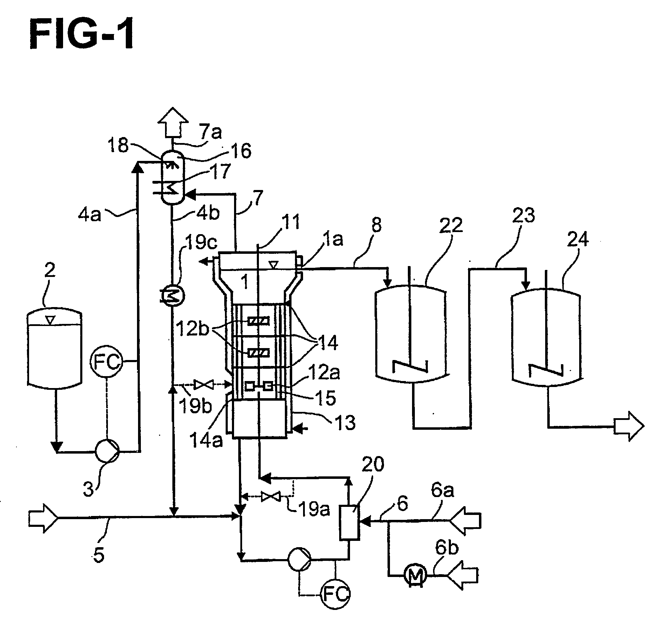

In der in Fig. 1 dargestellten Anlage erfolgt die Reaktion zur Herstellung von bevorzugt (cyclo)aliphatischen, Biuretgruppen enthaltenden Polyisocyanaten in einem Rührreaktor 1 und ein Mischkreis. Letzterem werden aus einem Behälter 2 über eine Pumpe 3 durch eine Leitung 4a/b Isocyanat, beispielsweise Hexamethylendiisocyanat (HDI) als einer der beiden Reaktanden zugeführt. Durch Leitung 5, die, wie gezeigt, mit Leitung 4b vereinigt oder von dieser getrennt in den Mischkreis geführt werden kann, können Katalysatoren eingeführt werden. In den Mischkreis wird durch Leitung 6 gegebenenfalls mit gasförmigem Stickstoff 6b verdünnter Wasserdampf 6a als zweiter Reaktand eingeführt. Die bei der Reaktion entstehenden Abgase, insbesondere CO₂, werden im Reaktor durch die Leitung 7 vom Kopf des Rührbehälters 1 abgeführt. Das bei der Reaktion entstehende Produkt, nämlich das Biuretgruppen aufweisende Polyisocyanat, wird dem oberen Ende des Behälters 1 durch Leitung 8 entnommen und über eine Pumpe 9 (nicht dargestellt) oder bevorzugt per Überlauf einem Auffangbehälter zugeführt.

Im Rührbehälter 1 sind um eine senkrechte Achse 11 drehbare Rührer 12 angeordnet. Die Temperatur im Rührbehälter 1 wird durch einen diesen umschließenden beheizbaren Mantel 13 eingestellt.

Der mit 1 bezeichnete Rührbehälter ist kaskadenförmig aufgebaut (kaskadierter Rührbehälter). In diesem Behälter sind an der parallel zur Längsachse des Behälters 1 verlaufenden Drehachse 11 ein Scheibenrührer 12a, sowie 2 durch Schrägblattrührer 12b gerührte Segmente. Zwischen ihnen befinden sich zwei Trennelemente 14 im Abstand voneinander angeordnet, die an der Wand des Rührbehälters 1 befestigt sind und eine kreisrunde Mittelöffnung aufweisen. Weiterhin befinden sich vor (am Reaktoreingang) und nach (am Reaktorausgang) den gerührten Segmenten noch jeweils durch Trennscheiben 14 und 14a abgetrennte, ungerührte Segmente (Beruhigungszonen).

In dem dargestellten Ausführungsbeispiel hat der Rührbehälter 1 einen Innendurchmesser von 682 mm. Die Öffnungen in den Stromstörscheiben können unterschiedliche Öffnungen haben. So hat die Öffnung in den Trennelemente 14 einen Durchmesser von 240 mm und in der Stromstörscheibe 14a von 480 mm. Die Scheibenrührer 12a, von denen einer in Fig. 2 dargestellt ist, können unterschiedliche Abmessungen haben. In der Regel werden Norm-Scheibenrührer verwendet. Im vorliegenden Falle hat die Scheibe des unteren Scheibenrührers 12a einen Durchmesser von 210 mm. Der Außendurchmesser einschließlich der senkrechten Rührflächen 12A beträgt 280 mm. Die Rührflächen 12A sind rechteckig ausgebildet mit einer Höhe von 56 mm und einer Breite von 70 mm.

Die verwendeten Schrägblattrührer haben einen Gesamtdurchmesser von 340 mm mit sechs gleichmäßig um die Drehachse 11 angeordneten Blättern, die jeweils um einen Winkel von 45° gegen die Drehebene versetzt sind.

Im Inneren des Rührbehälters 1 sind vier Stromstörleisten 15 parallel zur Längsachse des Rührbehälters in einem Winkelabstand von je 90° bei einem Wandabstand von 14 mm angeordnet. Die Drehachse 11 mit den auf ihr angeordneten Rührern 12a und 12b dreht sich mit 60 bis 400 Umdrehungen pro Minute.

Dem Mischkreis wird aus dem Behälter 2 mit Hilfe der Pumpe 3 durch die Leitung 4a/b der Reaktand HDI zugeführt. Die Leitung 4 kann über einen Injektor 18 zunächst in einen Waschbehälter 16 führen, der einen Kühler 17 aufweist. Mit diesem Kühler kann das aus dem Rührbehälter 1 über die Leitung 7 ein- und durch die Leitung 7A abströmende Abgas gekühlt und kondensierbare Anteile auskondensiert werden. Eine Abscheidung von Rückständen in der Leitung 7 wird hierdurch verhindert. Aus dem oberen Teil des Kühlbehälters 16 strömt das im wesentlichen aus CO₂ bestehende Abgas durch Leitung 7a ab und kann beispielsweise in eine Verbrennung geleitet werden, gegebenenfalls nach einer alkalischen Wäsche.

Das durch die Leitung 6 in den Mischkreis geführte Wasserdampf-/Stickstoffgemisch wird in die Mischeinrichtung 20 geführt. Dies geschieht in der in Fig. 1 dargestellten Ausführungsform in der Weise, daß die Leitung 6 in eine Düse 20 als Mischorgan eintritt, in dem die Vermischung mit dem HDI/Katalysator-Gemisch erfolgt.

In der Mischvorrichtung (Düse) 20 wird das Biuretisierungsmittel Wasserdampf im Gemisch mit Stickstoff im Volumenverhältnis 1 : 0,5 über die Leitung 6 mit einem isocyanathaltigen Strom, in den zusätzlich Katalysator über die Leitung 5 zudosiert wird, mit einer Dispergierarbeit von 2 × 10⁶ W/kg Wasserdampf in einer konzentrischen Mischdüse vermischt, in der Isocyanat außen und Wasserdampf innen eingeführt wird. Bei dem isocyanathaltigen Strom kann es sich um reines Frischisocyanat handeln, das direkt aus dem Waschbehälter 16 stammt, oder um ein Reaktionsgemisch, das dem Rührbehälter 1 entnommen wird, bevorzugt aus einer Beruhigungszone zu Beginn des Reaktors, oder um eine Mischung aus Frischisocyanat aus Waschbehälter 16 mit einem Reaktionsgemisch aus Rührbehälter 1 (bevorzugte Ausführungsform) oder um ein im Kreislauf über die Leitung 19a (in Fig. 1 gestrichelt dargestellt) gefördertes Produkt. Das frisch zugeförderte Isocyanat und der Katalysator können zusammen oder getrennt in diesen Kreislauf eindosiert werden und/oder in den Reaktor gefördert werden (Leitung 19b, in Fig. 1 gestrichelt dargestellt). Das frisch zugeführte Isocyanat kann bei Bedarf in einem Wärmetauscher 19c vorgewärmt werden, bevorzugt auf Reaktionstemperatur.

Das Volumenverhältnis von im Kreis gefördertem isocyanathaltigen Strom zu frischem isocyanathaltigem Strom beträgt in der Regel von 10 -100 : 1, bevorzugt 10 - 50 : 1. Die Temperatur im Kreislauf beträgt in einer bevorzugten Ausführungsform 130 -145°C, besonders bevorzugt um 140 °C.

Das Produkt wird dem oberen Ende des Rührbehälters 1 bei 1A entnommen und kann per Überlauf über die Leitung 8 einem Auffangbehälter (nicht dargestellt) zugeleitet werden.

In einer besonders bevorzugten Ausführungsform der Erfindung kann die Anlage, wie in Fig. 1 dargestellt, mit mindestens einem Nachreaktor betrieben werden, indem das aus dem Reaktor 1 über die Leitung 8 herausgeförderte Produkt in einer weiteren Reaktionsstufe iii) in mindestens einem Nachreaktor 22 thermisch nachbehandelt wird. Diese Nachreaktion zeichnet sich gegenüber der Reaktion im Reaktor 1 in Stufe ii) dadurch aus, daß das bei der Reaktion entstandene Kohlenstoffdioxid bereits weitgehend aus dem Reaktionsgemisch abgetrennt ist, d.h. zu mindestens 80 Gew%, bevorzugt zu mindestens 85 Gew%, besonders bevorzugt zu mindestens 90, ganz besonders bevorzugt zu mindestens 95 und insbesondere zu mindestens 98 Gew%. Bei diesem Nachreaktor 22 kann es sich um einen einzelnen Rührreaktor oder um eine Kaskade aus mehreren, beispielsweise, wie dargestellt, 2 Rührreaktoren 22 und 24 handeln, aber auch um einen Rohrreaktor.

In der Nachreaktionszone wird das per Leitung 8 aus dem Reaktor 1 geförderte Produkt bei einer Temperatur von 80 bis 180 °C über eine Verweilzeit von 1 bis 4 Stunden thermisch nachbehandelt. Durch diese Maßnahme kann die Farbzahl und/oder die Lagerstabilität des biuretgruppenhaltigen Produktes verbessert werden, sowie ferner, falls erforderlich, die gewünschte Oligomerenverteilung und Viskosität eingestellt werden. Ferner wird noch im Reaktionsgemisch verbliebenes CO₂ in der Nachbehandlung ausgetrieben.

Die erfindungsgemäß hergestellten Produkte zeichnen sich durch eine verbesserte Lagerstabilität aus, was bedeutet, daß sich während einer mehrwöchigen Lagerung ein geringerer Teil an Monomeren aus dem biuretgruppenhaltigen Polyisocyanat rückspaltet, als aus dem Stand der Technik bekannt.

Die erfindungsgemäße Anlage kann kontinuierlich und halbkontinuierlich betrieben werden. Bei der halbkontinuierlichen Fahrweise wird HDI und Katalysator vorgelegt und aufgeheizt. Anschließend wird kontinuierlich das Gemisch aus Wasserdampf und Stickstoff eingeleitet. Die Umsetzung dauert dabei insgesamt ca. 3 bis 4 Stunden.

Bei der Durchlauf-Fahrweise wird kontinuierlich HDI und Katalysator in den Mischkreis dosiert und parallel dazu das Gemisch aus Wasserdampf und Stickstoff eingeleitet. Das Rohprodukt aus HDI-Biuret-Oligomeren und überschüssigem Monomeren wird kontinuierlich über die Leitung 8 ausgetragen. Das Rohprodukt wird anschließend mittels Destillation aufgearbeitet.

Im allgemeinen wird es, um Produkte, die bei der Verarbeitung keine gefährlichen Mengen an Isocyanaten freisetzen, zu erhalten, erforderlich sein, den größten Teil der unumgesetzten Isocyanate (a) von den gebildeten biuretgruppenhaltigen Polyisocyanaten abzutrennen. Meistens werden Produkte gewünscht, deren Gehalt an den monomeren Isocyanaten (a) weniger als 1, bevorzugt weniger als 0,75 Gew.-%, besonders bevorzugt weniger als 0,5 und ganz besonders bevorzugt weniger als 0,3 Gew%, bezogen auf die biuretgruppenhaltigen Polyisocyanate, beträgt. Die Abtrennung der Isocyanate (a) nimmt man günstigerweise bei vermindertem Druck bei Temperaturen vor, die zwischen 50°C und der bei der Umsetzung gewählten Reaktionstemperatur liegen, indem man sie beispielsweise abdestilliert, bevorzugt an Dünnschichtverdampfern.

Als Apparate dafür dienen Flash-, Fallfilm-, Dünnschicht- oder Kurzwegverdampfer, denen gegebenenfalls eine kurze Kolonne aufgesetzt sein kann.

Die Destillation erfolgt in der Regel bei einem Druck zwischen 0,1 und 300 hPa, bevorzugt unter 200 hPa und besonders bevorzugt unter 100 hPa.

Mit dem erfindungsgemäß beschriebenen Verfahren werden in der Regel Produkte erhalten, die eine Farbzahl von weniger als 20 APHA gemäß DIN ISO 6271 und/oder eine Viskosität von 1000 bis 10000 mPas gemäß DIN 53019 Teil 1 (Rotationsviskosimeter) aufweisen.

Neben biuretgruppenhaltigen Polyisocyanaten können auch in untergeordnetem Maße uretdion- und/oder allophanatgruppenhaltige Polyisocyanate enthalten sein. Der Anteil an derartigen Polyisocyanaten beträgt jeweils weniger als 5 Gew%, besonders bevorzugt weniger als 3, ganz besonders bevorzugt weniger als 2, insbesondere weniger als 1 und speziell weniger als 0,5 Gew%.

In der Lackindustrie werden vor allem biuretgruppen-haltige Polyisocyanate, die eine Viskosität von 2.000 bis 20.000, bevorzugt von 2.500 bis 15.000 mPa·s und besonders bevorzugt 3.000 bis 12.000 mPa·s (bezogen auf einen Feststoffgehalt von 100% gemessen bei einer Temperatur von 23°C und einem Schergefälle von 100 s⁻¹) aufweisen, gewünscht. Derartige Polyisocyanate können bei Bedarf mit Lösungsmitteln verdünnt werden, beispielsweise den oben angeführten, bevorzugt Butylacetat, Xylol oder Methoxypropylacetat.

Die nach dem erfindungsgemäßen Verfahren erhaltenen Produkte eignen sich insbesondere als Härter in der Lackindustrie. Die Verarbeitung dieser Härter zu Lacken und hieraus hergestellten Beschichtungen ist allgemein bekannt.

### Beispiele

In der in den Figuren 1 und 2 dargestellten und oben beschriebenen Anlage wurde unter Umgehung der Leitungen 19a und 19b 1,6-Hexamethylendiisocyanat (HDI) bei 140°C in Gegenwart eines Wasserdampf/Stickstoff-Gemisches biuretisiert. Die mittlere Verweilzeit im Reaktor 1 betrug ca. 4 Stunden. Pro 1000 kg HDI wurden 4 kg Katalysator zugesetzt.

Das so erhaltene Reaktionsgemisch wurde dann in über weitere ca. 4 Stunden in den Behältern 22 und 24 bei etwa 140°C nachbehandelt und anschließend destillativ unumgesetztes HDI entfernt, so daß man den unten angegeben Gehalt an freiem HDI erhielt.

Ein erfindungsgemäß hergestelltes, biuretgruppenhaltige Polyisocyanat auf Basis 1,6-Hexamethylendiisocyanat (HDI) wurde bei Raumtemperatur über mehrere Monate gelagert und der Gehalt an freiem HDI (gaschromatographisch gemäß DIN 55956) bestimmt.

Man fand folgende Werte:

| | freies HDI [Gew%] |
|---|---|
| Vor Lagerung | 0,28 |
| nach 3 Monaten | 0,28 |
| nach 6 Monaten | 0,28 |

Die Meßreihe wurde wiederholt bei einer Lagertemperatur von 50 °C:

| | freies HDI [Gew%] |
|---|---|
| vor Lagerung | 0,10 |
| nach 1 Wochen | 0,09 |
| nach 2 Wochen | 0,15 |
| nach 3 Wochen | 0,16 |
| nach 4 Wochen | 0,17 |
| nach 2 Monaten | 0,20 |
| nach 3 Monaten | 0,21 |
| nach 6 Monaten | 0,38 |
| nach 9 Monaten | 0,30 |

Eine Durchführung analog der DE-A1 195 25 474 erbrachte während der Lagerung einen höheren Gehalt an freiem HDI.

## Patentansprüche

1. Verfahren zur Herstellung von biuretgruppenhaltigen Polyisocyanaten aus
a) mindestens einem Di- und/oder Polyisocyanat,
b) Wasser und/oder Wasserdampf (Wasser(dampf)) als Biuretisierungsmittel,
c) sowie gegebenenfalls mindestens einem Katalysator unter Freisetzung von Kohlenstoffdioxid, umfassend die Reaktionsschritte
i) Vermischen der Komponenten a) und b) sowie gegebenenfalls c) in einer Mischeinrichtung mit einer Mischenergie von mindestens 0,05 × 10⁶ J/kg Wasser(dampf) und
ii) Leiten des aus i) erhaltenen Reaktionsgemisches in mindestens einen Reaktor, in dem das Reaktionsgemisch, Wasser(dampf) und Kohlenstoffdioxid im Gleichstrom oder Gegenstrom geführt werden.

2. Verfahren nach Anspruch 1, **dadurch gekennzeichnet, daß** das Reaktionsgemisch, Wasser(dampf) und Kohlenstoffdioxid im Gleichstrom geführt werden.

3. Verfahren nach einem der vorstehenden Ansprüche, **dadurch gekennzeichnet, daß** die Temperatur im Schritt ii) zwischen 100 und 190 °C beträgt.

4. Verfahren nach einem der vorstehenden Ansprüche, **dadurch gekennzeichnet, daß** die mittlere Verweilzeit im Schritt ii) zwischen 0,4 und 6 Stunden beträgt.

5. Verfahren nach einem der vorstehenden Ansprüche, **dadurch gekennzeichnet, daß** die Mischzeit in der Mischeinrichtung 0,05 bis 60 s beträgt.

6. Verfahren nach einem der vorstehenden Ansprüche, **dadurch gekennzeichnet, daß** man das Reaktionsgemisch aus Schritt ii) nach Abtrennen von mindestens 80% des enthaltenen Kohlenstoffdioxids bei einer Temperatur von 80 bis -180 °C über eine Verweilzeit von 1 bis 4 Stunden thermisch nachbehandelt.

7. Vorrichtung, umfassend
- mindestens eine Mischeinrichtung
- in einem Umpumpkreislauf
- in den über Zuleitungen direkt oder vereinigt Isocyanat, Katalysator, Wasser(dampf) sowie gegebenenfalls Inertgas geführt werden können,
- aus dessen Kreislauf im Kreis gefördertes Produkt in einen nachgeschalteten kaskadierten Rührreaktor mit 2 bis 10 gerührten Segmenten gefördert wird,
- gefolgt von mindestens einer Beruhigungszone zur gas-flüssig-Trennung.

8. Vorrichtung nach Anspruch 7, zusätzlich aufweisend
- mindestens einen Nachreaktor, in den die Flüssigphase aus der Beruhigungszone geleitet werden kann.

9. Verwendung einer Vorrichtung gemäß Anspruch 7 bis 8 zur Herstellung von biuretgruppenhaltigen Polyisocyanaten.

## Claims

1. A process for the preparation of polyisocyanates containing biuret groups from
a) at least one di- and/or polyisocyanate,
b) water and/or steam (water (vapor)) as a biuretizing agent,
c) and, if appropriate, at least one catalyst with liberation of carbon dioxide, comprising the reaction steps
i) mixing of the components a) and b) and, if appropriate, c) in a mixing means with a mixing energy of at least 0.05 × 10⁶ J/kg of water (vapor) and
ii) passing of the reaction mixture obtained from i) into at least one reactor in which the reaction mixture, water (vapor) and carbon dioxide are passed cocurrently or countercurrently.

2. The process according to claim 1, wherein the reaction mixture, water (vapor) and carbon dioxide are passed cocurrently.

3. The process according to any of the preceding claims, wherein the temperature in step ii) is from 100 to 190°C.

4. The process according to any of the preceding claims, wherein the average residence time in step ii) is from 0.4 to 6 hours.

5. The process according to any of the preceding claims, wherein the mixing time in the mixing apparatus is from 0.05 to 60 s.

6. The process according to any of the preceding claims, wherein, after at least 80% of the carbon dioxide comprised have been separated off, the reaction mixture from step ii) is thermally aftertreated at a temperature of from 80 to 180°C for a residence time of from 1 to 4 hours.

7. An apparatus comprising
- at least one mixing means
- in a pumped circulation
- into which isocyanate, catalyst, water (vapor) and, if appropriate, inert gas can be passed directly or in combination via feed pipes,
- from whose circulation circulated product is transported to a downstream cascaded stirred reactor having from 2 to 10 stirred segments,
- followed by at least one calming zone for gas-liquid separation.

8. The apparatus according to claim 7, additionally having
- at least one downstream reactor into which the liquid phase from the calming zone can be passed.

9. The use of an apparatus according to claims 7 and 8 for the preparation of polyisocyanates containing biuret groups.

## Revendications

1. Procédé de préparation de polyisocyanates contenant des groupes biuret, constitués de :
a) au moins un diisocyanate et/ou un polyisocyanate,
b) eau et/ou vapeur d'eau en tant qu'agent de biurétisation,
c) ainsi qu'éventuellement au moins un catalyseur avec libération de dioxyde de carbone,
comprenant les étapes réactionnelles consistant à :
i) mélanger les composants a) et b) ainsi qu'éventuellement c) dans un dispositif de mélange ayant une énergie de mélange d'au moins 0,05 × 10⁶ J/kg d'eau et/ou de vapeur d'eau et
ii) diriger le mélange réactionnel obtenu à partir de i) dans au moins un réacteur, dans lequel le mélange réactionnel, l'eau et/ou la vapeur d'eau et le dioxyde de carbone sont amenés en courant parallèle ou à contre-courant.

2. Procédé selon la revendication 1, **caractérisé en ce que** le mélange réactionnel, l'eau et/ou la vapeur d'eau et le dioxyde de carbone sont amenés en courant parallèle.

3. Procédé selon l'une quelconque des revendications précédentes, **caractérisé en ce que** la température dans l'étape ii) est comprise entre 100 et 190 °C.

4. Procédé selon l'une quelconque des revendications précédentes, **caractérisé en ce que** le temps de séjour moyen dans l'étape ii) est compris entre 0,4 et 6 heures.

5. Procédé selon l'une quelconque des revendications précédentes, **caractérisé en ce que** le temps de mélange dans le dispositif de mélange est de 0,05 à 60 s.

6. Procédé selon l'une quelconque des revendications précédentes, **caractérisé en ce que** le mélange réactionnel issu de l'étape ii) est retraité de manière thermique après séparation d'au moins 80 % du dioxyde de carbone contenu à une température de 80 à 180 °C avec un temps de séjour de 1 à 4 heures.

7. Dispositif, comprenant :
- au moins un dispositif de mélange
- dans un circuit de transvasage
- dans lequel de l'isocyanate, du catalyseur, de l'eau et/ou de la vapeur d'eau ainsi qu'éventuellement du gaz inerte peuvent être amenés de manière directe ou réunie par l'intermédiaire de conduites,
- du produit en circulation provenant de ce circuit est transporté dans un réacteur agitateur placé en aval en cascade et ayant 2 à 10 secteurs agités,
- ce qui est suivi par au moins une zone de tranquillisation pour la séparation gaz-liquide.

8. Dispositif selon la revendication 7, présentant de manière supplémentaire :
- au moins un réacteur secondaire, dans lequel la phase liquide en provenance de la zone de tranquillisation peut être dirigée.

9. Utilisation d'un dispositif selon la revendication 7 à 8 en vue de la préparation de polyisocyanates contenant des groupes biuret.
